(19) Europäisches Patentamt
European Patent Office
Office européen des brevets

(11) Numéro de publication : **0 377 381 B1**

# (12) FASCICULE DE BREVET EUROPEEN

(45) Date de publication du fascicule du brevet : **22.04.92 Bulletin 92/17**

(51) Int. Cl.$^5$ : **C07D 209/49**

(21) Numéro de dépôt : **89403659.9**

(22) Date de dépôt : **27.12.89**

## (54) Procédé de préparation du phényl-1 diéthylaminocarbonyl-1 phtalimidométhyl-2 cyclopropane Z.

(30) Priorité : **28.12.88 FR 8817326**

(43) Date de publication de la demande : **11.07.90 Bulletin 90/28**

(45) Mention de la délivrance du brevet : **22.04.92 Bulletin 92/17**

(84) Etats contractants désignés : **AT BE CH DE ES GB GR IT LI LU NL SE**

(56) Documents cités : **EP-A- 0 200 638**

(73) Titulaire : **PIERRE FABRE MEDICAMENT**
**45, Place Abel Gance**
**F-92100 Boulogne (FR)**

(72) Inventeur : **Bigg, Dennis**
**122, Avenue de Lavaur**
**F-81100 Castres (FR)**
Inventeur : **Lesimple, Patrick**
**37 bis, Rue du Réservoir Lagarrigue**
**F-81090 Castres (FR)**

(74) Mandataire : **Ahner, Francis et al**
**CABINET REGIMBEAU, 26, avenue Kléber**
**F-75116 Paris (FR)**

EP 0 377 381 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention, réalisée au Centre de Recherche PIERRE FABRE MEDICAMENT, concerne un nouveau procédé de préparation du phényl-1 diéthylaminocarbonyl-1 phtalimidométhyl-2 cyclopropane Z répondant à la formule I.

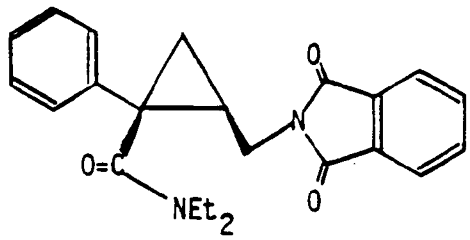

I

Le phényl-1 diéthylaminocarbonyl-1 phtalimidométhyl-2 cyclopropane Z de formule I sert de précurseur pour la fabrication du MILNACIPRAN (D.C.I.) un médicament utile dans le traitement de la dépression et qui répond à la formule II.

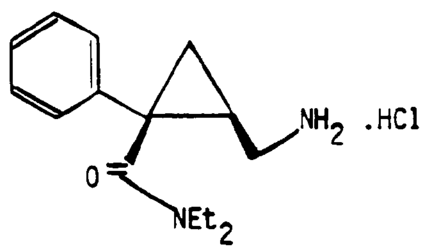

II

Dans la technique antérieure, illustrée par F 2.581.059, le composé de formule I est préparé en plusieurs étapes à partir du phényl-1 oxo-2 oxa-3 bicyclo (3:1:0) hexane avec un rendement d'environ 50 %.

L'objet de la présente invention concerne un nouveau procédé de préparation du composé de formule I avec un excellent rendement selon le schéma suivant :

**Première étape :**

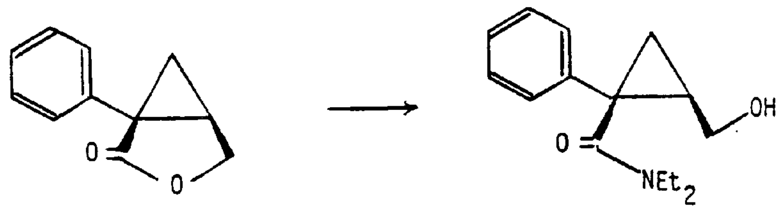

III IV

**Deuxième étape :**

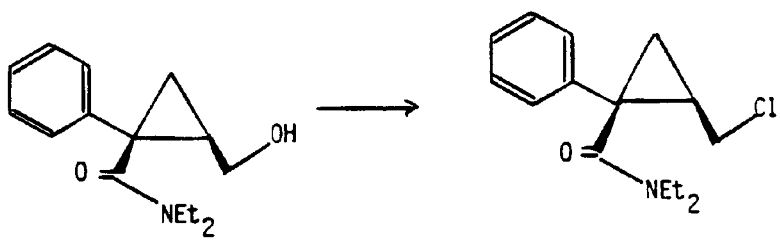

IV V

**Troisième étape :**

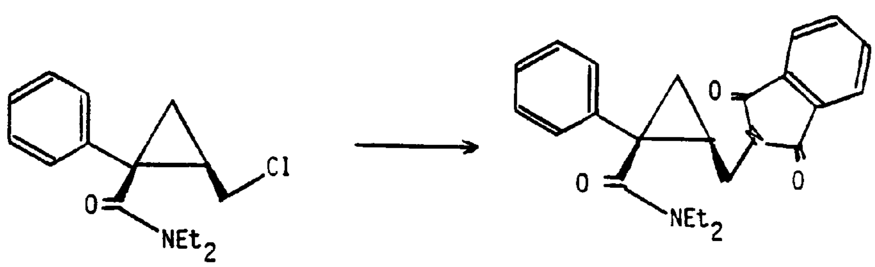

V I

La première étape du procédé consiste en l'ouverture de la lactone de formule II, avec la diéthylamine a l'aide d'un complexe acide de Lewis-amine au sein d'un solvant organique approprié tel que le dichloroéthane ou le dichlorométhane.

L'acide de Lewis employé est, de préférence, le chlorure d'aluminium, et l'amine du complexe est, soit la diéthylamine, soit une amine tertiaire. Parmi ces amines tertiaires, on peut citer à titre d'exemples non limitatifs, la triéthylamine, la diisopropyléthylamine, la N,N-diéthylaniline, la N,N-diméthylbenzylamine, la N-méthylpipéridine, la N-méthylmorpholine, la N,N'-diméthylpipérazine et la hexaméthylène tétramine.

La réaction est avantageusement conduite avec au moins un équivalent du complexe chlorure d'aluminium-amine, et, au moins un équivalent de la diéthylamine.

La réaction est avantageusement effectuée a une température comprise entre 10°C et 30°C.

Le rendement de cette première étape est presque quantitatif, ce qui permet d'obtenir un produit brut d'excellente qualité qui peut servir directement pour l'étape suivante.

Une purification ultérieure peut toutefois être effectuée si nécessaire par les moyens connus tels que la chromatographie ou la recristallisation, par exemple d'un mélange dichlorométhane/éther diisopropylique.

Au cours de la deuxième étape réactionnelle, le phényl-1 diéthylaminocarbonyl-1 hydroxyméthyl-2 cyclopropane Z de Formule IV est converti en phényl-1 diéthylaminocarbonyl-1 chlorométhyl-2 cyclopropane Z de Formule V à l'aide d'un réactif de chloration, tel que le chlorure de thionyle, de préférence utilisé en excès stoechiométrique.

La réaction est avantageusement conduite à une température comprise entre 10 et 50°C, de préférence à la température ambiante dans un solvant inerte tel que le dichloroéthane ou le dichlorométhane.

Le produit obtenu est de très bonne qualité et peut servir directement pour l'étape suivante.

Au cours de la troisième étape, on fait réagir le dérivé chloré de formule V avec un sel de phtalimide, en particulier le sel potassique, au sein d'un solvant organique tel que le diméthylformamide, le diméthylacétamide, la méthylpyrrolidone, le toluène, ou le dichloroéthane.

La température réactionnelle est, de préférence, comprise entre 80°C et 120°C.

Le phényl-1 diéthylaminocarbonyl-1 phtalimidométhyl-2 cyclopropane Z de formule I est obtenu avec un rendement élevé et le produit est d'excellente pureté.

Les rendements à chaque étape et la pureté des produits obtenus sont tels que le procédé décrit permet une synthèse industrielle économique d'une efficacité surprenante.

Les exemples suivants illustrent l'invention sans toutefois en limiter la portée.

Exemple 1 :

Phényl-1 diéthylaminocarbonyl-1 hydroxyméthyl-2 cyclopropane Z

(composé IV)

Méthode avec le complexe $AlCl_3$-$Et_2NH$

40 g de chlorure d'aluminium sont ajoutés avec agitation à une solution de 50 g de phényl-1 oxo-2 oxa-3 bicyclo (3:1:0) hexane dans 500 ml de dichloroéthane.

Le ballon est refroidi et on introduit 63 ml de diéthylamine dissoute dans du dichloroéthane. On maintient l'agitation pendant 0,5 heure, puis on hydrolyse par 1 l d'eau froide.

La phase organique est lavée à l'eau, à la saumure, et, enfin, séchée sur sulfate de sodium.

La phase organique ainsi séchée est évaporée a sec et on obtient 67,9 g de composé IV sous forme de cristaux crème.

| | |
|---|---|
| Rendement : | 95 % |
| Pureté (HPLC) : | 97 % |
| Point de fusion : | 54-55°C |

Exemple 2 :

Phényl-1 diéthylaminocarbonyl-1 hydroxyméthyl-2 cyclopropane Z

(composé IV)

Méthode avec le complexe $AlCl_3$-$Et_3N$

On ajoute sous agitation 4,2 ml de triéthylamine avec refroidissement à une suspension de 3,5 g de chlorure d'aluminium dans 20 ml de dichloroéthane.

Lorsque le milieu réactionnel est homogène, on ajoute 3,5 g de la lactone dissoute dans 20 ml de dichloroéthane et ensuite on ajoute 2,5 ml de diéthylamine.

Le mélange réactionnel est agité pendant 3 h à température ambiante et ensuite traité selon la méthode décrite en exemple 1 pour fournir 4,35 g du composé IV sous forme de cristaux crèmes.

| | |
|---|---|
| Rendement : | 90 % |
| Point de Fusion : | 54-55°C |

Exemple 3 :

Phényl-1 diéthylaminocarbonyl-1 hydroxyméthyl-2 cyclopropane Z

(composé IV)

Méthode avec le complexe $AlCl_3$-N-méthylmorpholine

D'une façon similaire à celle décrite dans l'exemple 2, mais en utilisant la N-méthylmorpholine, on obtient 4,3 g du composé IV sous forme de cristaux crème.

Rendement : 87 %

Point de fusion : 54-55°C

Exemple 4 :

Phényl-1 diéthylaminocarbonyl-1 chlorométhyl-2 cyclopropane Z

(composé V)

A une solution de 15,5 g de phényl-1 diéthylaminocarbonyl-1 hydroxyméthyl-2 cyclopropane Z (composé IV) dans 100 ml de dichloroéthane sont ajoutés 5 ml de chlorure de thionyle. On agite le mélange réactionnel température ambiante pendant 0,25 h et on concentre à sec.

Le sirop obtenu est repris dans 100 ml de dichloroéthane et on lave successivement avec de l'eau, une solution de bicarbonate de sodium et, à nouveau, à l'eau.

On sèche la phase organique sur sulfate de sodium et, après évaporation, on obtient 16,2 g du composé V sous forme d'huile.

Rendement : 97 %.

CCM (gel de silice GF 245 Merck)

Rf : 0,8 (hexane/acétate d'éthyle : 2/3)

IR : 1635 $cm^{-1}$ ($C(O)NEt_2$)

Exemple 5 :

Phényl-1 diéthylaminocarbonyl-1 phthalimidométhyl-2-cyclopropane Z

(composé I)

10 g de phényl-1 diéthylaminocarbonyl-1 chlorométhyl-2 cyclopropane Z (composé V) sont dissous dans 20 ml de DMF, puis on ajoute 8,7 g de phtalimide potassique et le milieu réactionnel est chauffé trois heures à 110°C.

On laisse refroidir et on verse la solution dans 150 ml d'eau.

Après filtration, lavage à l'eau et séchage, on obtient 13,5 g de composé I sous forme de cristaux blancs.

Rendement : 95%

Pureté (HPLC) : 99 %

Point de fusion : 131-132°C

Exemple 6 :

Phényl-1 diéthylaminocarbonyl-1 phtalimidométhyl-2 cyclopropane Z

(composé I)

Procédé dans isolement des intermédiaires.

Un réacteur de 20 l est chargé avec 3,5 l de dichloroéthane et 510 g de chlorure d'aluminium. Une solution de 765 ml de diéthylamine dans 0,8 l de dichloroéthane est ajoutée avec refroidissement et sous agitation. Lorsque le mélange est homogène, on ajoute 500 g de phényl-1 oxo-2 oxa-3 bicyclo (3:1:0) hexane dissout dans 1,7 l de dichloroéthane.

La suspension qui se forme est agitée 2 h à température ambiante et, ensuite, on hydrolyse par l'addition de 9 l d'eau. Après décantation, la phase aqueuse est extraite avec du dichloroéthane et les phases organiques sont réunies et lavées par 8 l d'eau avant d'être séchés par distillation azéotropique.
La solution du phényl-1 diéthylaminocarbonyl-1 hydroxyméthyl-2 cyclopropane Z (composé IV) ainsi obtenue est concentrée jusqu'à 2,5 l environ et utilisée directement pour l'étape suivante.

La solution du composé IV dans le dichloroéthane obtenu selon la méthode ci-dessus est chargée dans un réacteur de 6 l et 215 ml de chlorure de thionyle sont ajoutés en maintenant la température a 25°C environ. Lorsque l'addition du chlorure de thionyle est terminée, on concentre le milieu sous vide, on ajoute 0,5 l de toluène et on distille pour entraîner les traces d'acide chlorhydrique.

On ajoute 3 l de diméthylformamide au phényl-1 diéthylaminocarbonyl-1 chlorométhyl-2 cyclopropane Z (composé V) ainsi obtenu, et, ensuite, 678 g de phtalimide potassique. Le mélange réactionnel est chauffé avec agitation pendant 2 h a 100-110°C. On laisse refroidir le milieu réactionnel puis on le verse dans 15 l d'eau avec agitation.

Les cristaux blanc crème obtenus sont filtrés et lavés a l'eau et, ensuite, le gâteau est réempâté dans le minimum d'éther diisopropylique. Après filtration et séchage, on obtient 980 g du phényl-1 diéthylaminocarbonyl-1 phtalimidométhyl-2 cyclopropane Z (composé I) sous forme de cristaux blancs.
Rendement global sur les trois étapes : 91 %
Pureté (HPLC) : ≧ 98 %
Point de fusion : 131-132° C

## Revendications

1. Procédé de préparation du phényl-1 diéthylaminocarbonyl-1 phtalimidométhyl-2 cyclopropane Z de formule I :

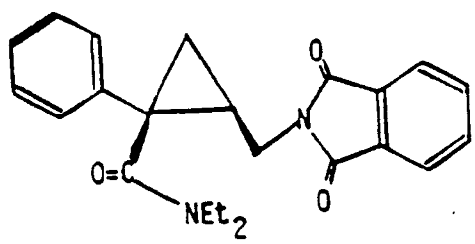

I

caractérisé par la mise en oeuvre des réactions successives suivantes :

– ouverture de la lactone phényl-1 oxo-2 oxa-3 bicyclo (3:1:0) hexane de formule III par la diéthylamine à l'aide d'un complexe d'acide de Lewis et d'une amine choisie parmi la diéthylamine et les amines tertiaires;

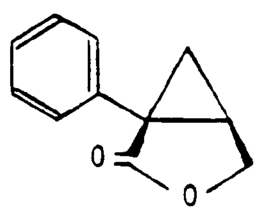

III

– le phényl-1 diéthylaminocarbonyl-1 hydroxyméthyl-2 cyclopropane Z de formule IV ainsi obtenu :

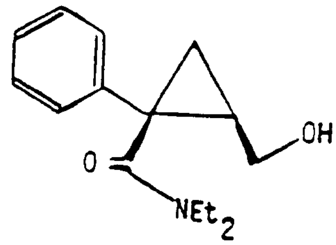

IV

est converti en dérivé chloré par l'action d'un réactif de chloration, tel que le chlorure de thionyle ;
– le phényl-1 diéthylaminocarbonyl-1 chlorométhyl-2 cyclopropane Z de formule V ainsi obtenue :

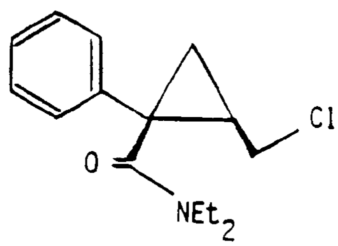

V

est ensuite traité par un sel de phtalimide au sein d'un solvant organique approprié pour obtenir le composé de formule I précité.

2. Procédé selon la revendication 1 caractérisé en ce que l'acide de Lewis utilisé est le chlorure d'aluminium.

3. Procédé selon l'une des revendications 1 et 2 caractérisé en ce que l'on emploie au moins un équivalent du complexe chlorure d'aluminium-amine.

4. Procédé selon l'une des revendications 1 à 3 caractérisé en ce que l'on emploie au moins un équivalent de la diéthylamine.

5. Procédé selon l'une des revendications 1 à 4 caractérisé en ce que le solvent employé pour l'ouverture de la lactone de formule III est choisi parmi le dichloroéthane et le dichlorométhane.

6. Procédé selon l'une des revendications 1 à 5 caractérisé en ce que la température réactionnelle est comprise entre 10°C et 30°C lors de l'ouverture de la lactone de formule III.

7. Procédé selon l'une des revendications 1 à 6 caractérisé en ce que l'on utilise le chlorure de thionyle lors de la deuxième étape pour effectuer la transformation de l'alcool de formule IV en dérivé chlore de formule V.

8. Procédé selon l'une des revendications 1 à 7 caractérisé en ce que la réaction de l'alcool de formule IV avec le chlorure de thionyle s'effectue à une température comprise entre 10°C et 50°C, de préférence à la température ambiante.

9. Procédé selon l'une des revendications 1 à 8 caractérisé en ce que la réaction entre l'alcool de formule IV et le chlorure de thionyle est effectué au sein d'un solvant organique tel que le dichloroéthane ou le dichlorméthane.

10. Procédé selon l'une des revendications 1 à 9 caractérisé en ce que le sel de phtalimide utilisé est le phtalimide de potassium.

11. Procédé selon l'une des revendications 1 à 10 caractérisé en ce que la réaction entre le composé chloré de formule V et le phtalimide de potassium est effectuée au sein d'un solvant organique choisi parmi le diméthylformamide, le diméthylacétamide, la méthylpyrrolidone, le toluène, et le dichloroéthane.

12. Procédé selon l'une des revendications 1 à 11 caractérisé en ce que la réaction entre le composé chloré de formule V et le phtalimide de potassium est effectuée à une température comprise entre 80°C et 120°C.

**Patentansprüche**

1. Verfahren zur Herstellung von 1-Phenyl-1-diethylaminocarbonyl-2-phthalimidomethyl-cyclopropan Z der Formel I :

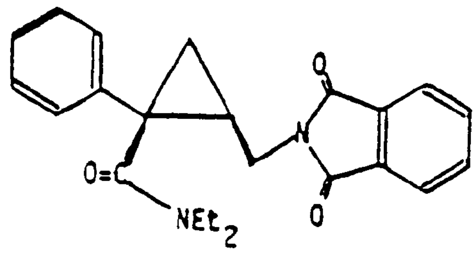

I

dadurch gekennzeichnet, daß man die folgenden Reaktionen nacheinander durchführt:

– Öffnen des 1-Phenyl-2-oxo-3-oxa-bicyclo(3:1:0)hexan-lactons der Formel III mit Diethylamin, mit Hilfe eines Komplexes von Lewis-Säure und einem Amin, ausgewählt aus Diethylamin und den tertiären Aminen;

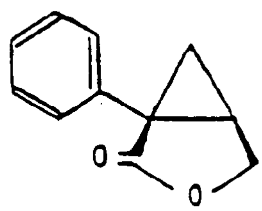

III

– Umwandeln des so erhaltenen 1-Phenyl-1-diethylaminocarbonyl-2-hydroxymethyl-cyclopropan Z der Formel IV :

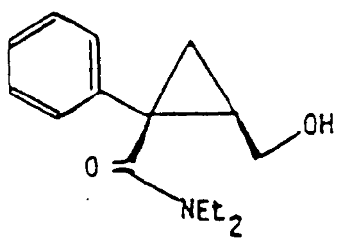

IV

in ein Chlorderivat durch Einwirken eines Chlorierungs-Reagens, wie Thionylchlorid;

– anschließendes Behandeln des so erhaltenen 1-Phenyl-1-diethylaminocarbonyl-2-chlormethyl-cyclopropan Z der Formel V :

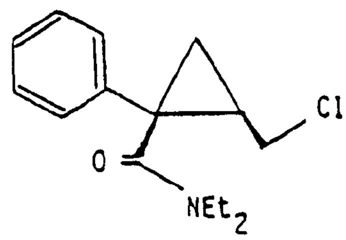

V

mit einem Phthalimidsalz in einem geeigneten organischen Lösungsmittel, um die Verbindung der vorstehenden Formel I zu erhalten.

2. Verfahren nach Anspruch 1, dadurch gekennzeichnet, daß die verwendete Lewis-Säure Aluminiumchlorid ist.

3. Verfahren nach einem der Ansprüche 1 und 2, dadurch gekennzeichnet, daß man mindestens ein Äquivalent des Aluminiumchlorid-Amin-Komplexes verwendet.

4. Verfahren nach einem der Ansprüche 1 bis 3, dadurch gekennzeichnet, daß man mindestens ein Äquivalent des Diethylamins verwendet.

5. Verfahren nach einem der Ansprüche 1 bis 4, dadurch gekennzeichnet, daß man das zur Öffnung des Lactons der Formel III verwendete Lösungsmittel aus Dichlorethan und Dichlormethan auswählt.

6. Verfahren nach einem der Ansprüche 1 bis 5, dadurch gekennzeichnet, daß die Reaktionstemperatur bei der Öffnung des Lactons der Formel III bei 10°C bis 30°C liegt.

7. Verfahren nach einem der Ansprüche 1 bis 6, dadurch gekennzeichnet, daß man bei der zweiten Stufe zur Umwandlung des Alkohols der Formel IV in ein Chlorderivat der Formel V Thionylchlorid verwendet.

8. Verfahren nach einem der Ansprüche 1 bis 7, dadurch gekennzeichnet, daß die Reaktion des Alkohols der Formel IV mit dem Thionylchlorid bei einer Temperatur von 10°C bis 50°C, vorzugsweise bei Raumtemperatur, durchgeführt wird.

9. Verfahren nach einem der Ansprüche 1 bis 8, dadurch gekennzeichnet, daß die Reaktion zwischen dem Alkohol der Formel IV und dem Thionylchlorid in einem organischen Lösungsmittel, wie Dichlorethan oder Dichlormethan durchgeführt wird.

10. Verfahren nach einem der Ansprüche 1 bis 9, dadurch gekennzeichnet, daß das verwendete Phthalimidsalz Kalium-phthalimid ist.

11. Verfahren nach einem der Ansprüche 1 bis 10, dadurch gekennzeichnet, daß die Reaktion zwischen der chlorierten Verbindung der Formel V und dem Kalium-phthalimid in einem organischen Löungsmittel, ausgewählt aus Dimethylformamid, Dimethylacetamid, Methylpyrrolidon, Toluol und Dichlorethan, durchgeführt wird.

12. Verfahren nach einem der Ansprüche 1 bis 11, dadurch gekennzeichnet, daß die Reaktion zwischen der chlorierten Verbindung der Formel V und dem Kalium-phthalimid bei einer Temperatur von 80°C bis 120°C durchgeführt wird.

## Claims

1. A process for the preparation of 1-phenyl 1-diethylaminocarbonyl 2-phthalimidomethyl cyclopropane Z of formula I:

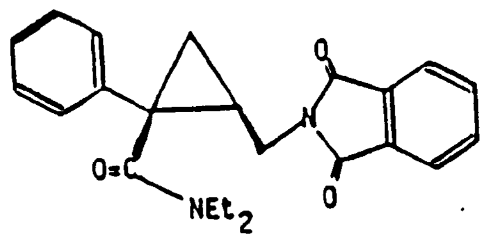

I

characterised by the use of the following successive reactions:

– opening of the lactone 1-phenyl 2-oxo 3-oxa bicyclo (3:1:∅) hexane of formula III by diethylamine by means of a complex of Lewis acid and an amine selected from diethylamine and the tertiary amines;

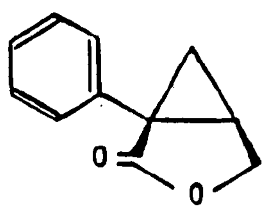

III

– the 1-phenyl 1-diethylaminocarbonyl 2-hydroxymethyl cyclopropane Z of formula IV thus obtained:

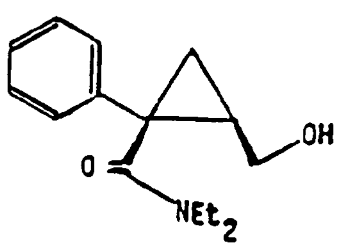

IV

is converted into a chlorinated derivative by the action of a chlorination reagent such as thionyl chloride;

– the 1-phenyl 1-diethylaminocarbonyl 2-chloromethyl cyclopropane Z of formula V thus obtained

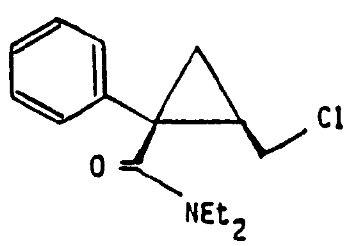

V

is then treated by phthalimide salt within a suitable organic solvent to give the above formula I compound.

2. A process according to claim 1, characterised in that the Lewis acid used is aluminium chloride.

3. A process according to claim 1 or 2, characterised in that at least one equivalent of the amine-aluminium chloride complex is used.

4. A process according to any one of claims 1 to 3, characterised in that at least one equivalent of diethylamine is used.

5. A process according to any one of claims 1 to 4, characterised in that the solvent used to open the formula III lactone is selected from dichloroethane and dichloromethane.

6. A process according to any one of claims 1 to 5, characterised in that the reaction temperature is contained between 10°C and 30°C during opening of the formula III lactone.

7. A process according to any one of claims 1 to 6, characterised in that the thionyl chloride is used during the second step to effect the conversion of the formula IV alcohol into a formula V chlorinated derivative.

8. A process according to any one of claims 1 to 7, characterised in that the reaction of the formula IV alcohol with thionyl chloride is carried out at a temperature of between 10°C and 50°C, preferably at ambient temperature.

9. A process according to any one of claims 1 to 8, characterised in that the reaction between the formula IV alcohol and the thionyl chloride is carried out within an organic solvent such as dichloroethane or dichloromethane.

10. A process according to any one of claims 1 to 9, characterised in that the phthalimide salt used is potassium phthalimide.

11. A process according to any one of claims 1 to 10, characterised in that the reaction between the formula V chlorinated compound and the potassium phthalimide is carried out within an organic solvent selected from dimethylformamide, dimethylacetamide, methylpyrrolidone, toluene and dichloroethane.

12. A process according to any one of claims 1 to 11, characterised in that the reaction between the formula V chlorinated compound and the potassium phthalimide is carried out at a temperature of between 80°C and 120°C.